# EUROPÄISCHE PATENTSCHRIFT

(11) **EP 3 899 536 B1**
(45) Veröffentlichungstag und Bekanntmachung des Hinweises auf die Patenterteilung: **10.08.2022**
(21) Anmeldenummer: 19832930.2
(22) Anmeldetag: 19.12.2019
(51) Int. Cl.: G01N 33/569, G01N 33/50, C07K 16/28

(54) **VERFAHREN ZUR SELEKTION BIOLOGISCHER BINDEMOLEKÜLE**
METHOD FOR SELECTING BIOLOGICAL BINDING MOLECULES
PROCÉDÉ DE SÉLECTION DE MOLÉCULES DE LIAISON BIOLOGIQUES

(30) Priorität: 20.12.2018 EP 18000989
(43) Veröffentlichungstag der Anmeldung: 27.10.2021
(73) Patentinhaber: AVA Lifescience GmbH, 79211 Denzlingen (DE)
(72) Erfinder: KLAPPROTH, Holger, 79108 Freiburg (DE); DÜHREN-VON MINDEN, Marcus, 79379 Müllheim (DE)
(74) Vertreter: Cacace, Sabrina Rosella
(86) Internationale Anmeldenummer: PCT/EP2019/086450
(87) Internationale Veröffentlichungsnummer: WO 2020/127827

(56) Entgegenhaltungen:
- EP-A1- 1 001 020
- EP-A1- 3 543 697
- US-B2- 9 926 381
- MARCUS DÜHREN-VON MINDEN ET AL: "Chronic lymphocytic leukaemia is driven by antigen-independent cell-autonomous signalling", NATURE, Bd. 489, Nr. 7415, 12. August 2012 (2012-08-12), Seiten 309-312, XP055481577, London ISSN: 0028-0836, DOI: 10.1038/nature11309
- BOJARCZUK KAMIL ET AL: "B-cell receptor signaling in the pathogenesis of lymphoid malignancies", BLOOD CELLS, MOLECULES AND DISEASES, LAJOLLA, US, Bd. 55, Nr. 3, 11. Juli 2015 (2015-07-11), Seiten 255-265, XP029252301, ISSN: 1079-9796, DOI: 10.1016/J.BCMD.2015.06.016
- GUR YAARI ET AL: "Practical guidelines for B-cell receptor repertoire sequencing analysis", GENOME MEDICINE, Bd. 7, Nr. 1, 20. November 2015 (2015-11-20), XP055453727, DOI: 10.1186/s13073-015-0243-2

## Beschreibung

Die vorliegende Erfindung betrifft das Gebiet der Herstellung, Identifizierung und Selektion von biologischen Bindemolekülen wie insbesondere von Antikörpern oder Fragmenten derselben, die selektiv an somatisch hypermutierte B-Zell Rezeptoren oder B-Zell Rezeptorkomplexe binden.

Als Rezeptor (von lateinisch recipere 'aufnehmen' bzw. 'empfangen') wird in der Biochemie ein Protein oder ein Proteinkomplex bezeichnet, wenn daran Signalmoleküle binden können, die durch das Bindungsereignis im Zellinneren Signalprozesse auszulösen vermögen. Ein Rezeptor kann Signale von außerhalb empfangen und sich an der Oberfläche einer Biomembran befinden, oder er kann im Cytosol der Zelle nachweisbar sein. Rezeptoren besitzen eine spezifische Bindungsstelle für ihren physiologischen Agonisten oder Antagonisten (Bindungspartner, Ligand).

Membranrezeptoren befinden sich an der Oberfläche von Biomembranen und bestehen aus Proteinen, die häufig mit Modifikationen versehen sind (z. B. Kohlenhydratketten). Sie besitzen eine bestimmte Passform für kleine Moleküle, sogenannte Liganden, oder für Teile von größeren Molekülen, die an die Rezeptorstruktur binden, indem sie diese als komplementäre Struktur ergänzen (vereinfachend 'Schlüssel-Schloss-Prinzip' genannt).

Rezeptoren können somit der Aufnahme und Weiterleitung von Signalen dienen (Signaltransduktion), oder funktionell am Zusammenhalt von Zellen (Zelladhäsion), oder dem Transport von Stoffen in die Zelle (Membrantransport) beteiligt sein. Ferner können sie Virionen die Möglichkeit bieten, an die passende Wirtszelle anzudocken und sie zu infizieren.

Zu den für Zellkontakte wichtigen Membranrezeptoren gehören sowohl Zelladhäsionsmoleküle, die Zell-Zell-Kontakte vermitteln wie die Cadherine, Selectine und Immunglobuline, als auch solche, die an der Ausbildung von Zell-Matrix-Kontakten beteiligt sind und Zellen an der extrazellulären Matrix verankern wie die Integrine.

Membranrezeptoren kommen nicht nur auf der Plasmamembran, sondern auch auf Biomembranen der Organellen im Zellinneren vor. Während außen liegende Zellmembranrezeptoren die Zelle in Beziehungen zum Außenraum als ihrer Umgebung setzen, werden im Innenraum der Zelle einzelne Organellen über Rezeptoren in Beziehung zum Zytoplasma, Zytoskelett oder zueinander gesetzt.

Rezeptoren in der Zellmembran werden nach ihrer Wirkungsweise unterteilt in ionotrope und metabotrope Rezeptoren.

Ionotrope Rezeptoren stellen Ionenkanäle dar, die sich bei Bindung eines geeigneten Liganden mit höherer Wahrscheinlichkeit öffnen und dadurch die Leitfähigkeit der Membran ändern.

Metabotrope Rezeptoren hingegen bilden keine Kanäle oder Poren, sondern aktivieren bei Bindung ihres Liganden einen nachgeschalteten "Second Messenger" (z.B. ein G-Protein oder eine Proteinkinase) und modulieren damit intrazelluläre Signalkaskaden durch Konzentrationsänderungen von sekundären Botenstoffen, was mittelbar aber auch zu einer Veränderung der Membrandurchlässigkeit führen kann.

Für viele Rezeptoren existieren natürliche Liganden, die zur Aktivierung dieser Rezeptoren führen und eine 'Second Messenger'-Kaskade auslösen. Neben natürlichen Liganden gibt es auch Substanzen, die an den Rezeptor binden und diesen entweder aktivieren (Agonisten) oder inaktivieren (Antagonisten). Beispiele für Rezeptor-Agonisten sind z.B. Antigene einschließlich Allergene, Opioide, Nikotin, Salbutamol, Muskarin, Zytokine, und Neurotransmitter.

Einen in dieser Hinsicht besonderen Rezeptor stellt der B-Zell Rezeptor oder B-Zell Rezeptorkomplex (BCR) dar. Dieser BCR wird von B-Zellen exprimiert und stellt quasi einen membrangebundenen Antikörper dar. Der BCR wird in großer Vielfalt in reifenden B-Zellen gebildet. In der Regel ist dieser BCR vom Typ IgD oder IgM.

Die Entwicklung der B-Zellen findet beim Menschen und auch bei einigen anderen Säugern im Knochenmark bzw. in der fetalen Leber statt. Die Signale, die für das Entwicklungsprogramm notwendig sind, erhalten die sich entwickelnden Lymphozyten von so genannten Stromazellen. Bei der B-Zell-Entwicklung ist die Bildung eines funktionierenden B-Zell Rezeptors (die membrangebundene Form des 'Antikörpers') von entscheidender Bedeutung. Nur mit diesem Antigenrezeptor sind reife B-Zellen später in der Lage, fremde Antigene zu erkennen und durch die Bildung von entsprechenden Antikörpern an feindliche Strukturen zu binden. Die Antigenspezifität des Rezeptors wird durch die Verknüpfung bestimmter Gensegmente bestimmt. Die Segmente heißen V-, D- und J-Segmente, weshalb der Prozess als V(D)J-Rekombination bezeichnet wird. Dabei werden diese Segmente, die den Antigen-bindenden Teil des B-Zell-Rezeptors bilden, umgeordnet. Der gesamte Rezeptor besteht aus zwei identischen leichten Proteinketten und zwei identischen schweren Proteinketten, wobei die schweren mit den leichten Ketten jeweils über Disulfidbrücken miteinander verknüpft sind. Bei der VDJ-Rekombination werden zuerst die V-, D- und J-Segmente der schweren Kette des B-Zell Rezeptors verknüpft, danach die V- und J-Segmente der leichten Rezeptorkette. Nur wenn die Gene dabei erfolgreich umgeordnet werden, was als produktive Genumordnung bezeichnet wird, kann die Zelle in den jeweils nächsten Entwicklungsschritt übergehen.

B-Zellen, die während ihrer Ausreifung im Knochenmark auf körpereigene Antigene reagieren, sterben in den allermeisten Fällen durch Apoptose ab. Im Blut von gesunden Menschen lassen sich geringe Mengen an autoreaktiven Zellen, unter anderem gegen Thyreoglobulin oder auch Kollagen, nachweisen (Abul K. Abbas: *Diseases of Immunity* in Vinay Kumar, Abul K. Abbas, Nelson Fausto : *Robbins and Cotran* - *Pathologic Basis of Disease.* 7. Auflage. Philadelphia 2005, S. 224f).

Als somatische Hypermutation wird das Einfügen von Mutationen in die Antikörpergene einer reifenden B-Zelle bezeichnet. Dieses Ereignis findet in follikulären B-Zellen statt. Die somatische Hypermutation ist der Prozess, den eine B-Zelle durchläuft, um den affinitätsgereiften Zustand zu erreichen. Es handelt sich um einen wichtigen Schritt des adaptiven Immunsystems. Mit Hilfe des Enzyms 'Activation Induced Cytidine Deaminase' (AID oder auch AICDA) entstehen zufällige Veränderungen der DNA. AID desaminiert Cytosin in einzelsträngiger DNA zu Uridin. Das Enzym UNG entfernt Uracil und schafft eine abasische Stelle. Dort wird von APE1 ein Einzelstrangbruch erzeugt. In Konsequenz wird auf diese Weise der zellinterne Reparaturmechanismus aktiviert, durch welchen dann bei der Reparatur zufällig Sequenzveränderungen eingefügt werden. Dies kann dann auf Aminosäureebene zu einer Strukturänderung und damit bedingt zu einer Affinitätsänderung des B-Zell Rezeptors (BCR) führen. Dieser Prozess endet, wenn über bisher nicht geklärte Mechanismen die Affinität zum Antigen eine bestimmte Stärke erreicht hat.

Durch Selektion werden diejenigen Zellen ausgewählt, welche am besten das Antigen binden und es somit am effektivsten bekämpfen können. Die übrigen Zellen gehen zugrunde. Dieser Prozess, welcher in den Keimzentren der sekundären lymphatischen Organe (Milz, Lymphknoten) stattfindet, ermöglicht es dem Organismus, eine Vielzahl von verschiedenen Antikörpern zu produzieren, und sich somit an die sich im Zuge einer Immunevasion verändernden Pathogene anzupassen sowie die Affinität der Antikörper zum Antigen zu erhöhen. Dieser Schritt führt zur Entstehung einzigartiger B-Zell Populationen, die in der Regel mono- oder oligoklonal sind. Ist eine solche Population an einem Krankheitsgeschehen mit autoimmunem oder malignem Hintergrund beteiligt (egal ob ursächlich oder unterstützend), so kann das Krankheitsgeschehen durch die Entfernung dieser Zellen aus dem Körper positiv beeinflusst werden, indem die krankheitsbedingten Symptome reduziert werden oder komplett verschwinden. Um diese B-Zellen selektiv aus dem Körper zu entfernen, werden extrem spezifische Antikörper benötigt. Diese können dann z.B. als membranständiger Rezeptor, als Teil eines chimären Rezeptors (z.B. mittels CAR-T Zellen), oder als therapeutischer Antikörper in löslicher Form verwendet werden. Eine weitere Anwendungsform ist eine prätherapeutische Apharese, in dem der matrixgebundene Antikörper dazu verwendet wird, die malignen Zellen aus dem Blut zu separieren. Die Tumorlast des Patienten würde dadurch deutlich verringert, und das Immunsystem wird so in einem geringeren Maße durch das Abtöten der verbliebenen Zelle belastet.

Das Entfernen dieser spezifischen B-Zellen führt dazu, dass das Immunsystem nicht wie durch das Abtöten aller B-Zellen völlig lahmgelegt wird und die bisherigen Immunitäten (z.B. nach Infekten oder Impfungen) aufgehoben werden, sondern dass der Patient trotz Therapie über ein weiterhin einsatzfähiges Immunsystem verfügt.

Für die Generierung von Antikörpern gegen Rezeptoren werden für die Immunisierung von Mäusen in der Regel lösliche Versionen dieser Rezeptoren (in rekombinanter und aufgereinigter Form) verwendet. In seltenen Fällen können auch Peptidfragmente verwendet werden. Aus den in der Maus entstehenden spezifischen B-Zellen werden mittels der Hybridomtechnologie Antikörper produzierende Hybridom-Zellen geschaffen.

Diese Hybridomazellen produzieren Antikörper, die dann mittels ELISA oder mittels exprimierter Rezeptoren in Zellsystemen getestet werden. Dazu werden herkömmlich etablierte Zelllinien verwendet, da sich nur diese einfach kultivieren lassen. Dabei können Antikörper erzeugt werden, die relativ spezifisch an einen bestimmten Rezeptortyp (z.B. Anti-IgGl, Anti-IgE) binden. Hierbei kommt es jedoch häufig zu Kreuzreaktionen mit anderen Rezeptoren oder anderen Epitopen.

Für eine therapeutische Anwendung von BCR-Antikörpern ist es zumeist nicht ausreichend, nur einen Antikörper gegen den BCR im allgemeinen einzusetzen, da ein solcher Breitbandeinsatz erhebliche Nebenwirkungen auslösen kann. Vielmehr wäre es wünschenswert, einen Antikörper bereitzustellen, der selektiv an einen Rezeptor bindet, der eine Aktivierung wie insbesondere eine unerwünschte (pathophysiologische oder autonome) Aktivierung aufweist. Ein solcher Antikörper ist im Stand der Technik nicht bekannt und ein Verfahren zu dessen Herstellung oder Gewinnung durch Selektion existiert nicht.

Die Aufgabe der Erfindung liegt somit in der Bereitstellung eines Systems zur Herstellung bzw. Gewinnung und Identifizierung bzw. Selektion von biologischen Bindemolekülen wie insbesondere von Antikörpern oder funktionellen Fragmenten derselben, die hoch-selektiv an affinitätsgereifte BCRs binden, welche sich durch die hypersomatischen Mutationen von den keimbahnkodierten Sequenzen der BCR Komponenten unterscheiden. Hierdurch ist es möglich, gegen krankheitsspezifische B-Zellen Antikörper zu entwickeln, die im Rahmen einer individualisierten Therapie vorteilhaft eingesetzt werden können. Hierbei ist es wichtig, dass die Rezeptoren oder Rezeptorkomplexe, die im Rahmen der Selektion verwendet werden, eine korrekte Faltung aufweisen und somit bevorzugt funktional sind.

Nach einer bevorzugten Ausführungsform wird dabei die Funktionalität des BCR vor der Verwendung zur Selektion überprüft. Werden die Zellen vor der Messung mit 4-OH Tamoxifen induziert, so kann die Aktivierung des BCR durch die Messung des Calciumsignals nachgewiesen werden (Dühren von Minden et al. 2012, Nature 489, p309-313). Als Referenz zur spezifischen Selektion wird ein BCR konstruiert, der dem Ziel-BCR ohne die somatischen Hypermutationen entspricht.

Die Aufgabe wird gelöst durch die Bereitstellung eines Verfahrens gemäß Hauptanspruch. Bevorzugte Ausführungsformen sind Gegenstand entsprechender Unteransprüche.

Bevor auf die einzelnen Aspekte der vorliegenden Erfindung detailliert eingegangen wird, erfolgt eine Klärung relevanter Begriffe, die im Rahmen der vorliegenden Beschreibung verwendet werden.

B-Zell Rezeptoren vom Keimbahntyp, d.h. BCR, deren Aminosäuresequenz von Gensequenzen kodiert werden, wie sie im Genom der Zelle vorkommen und somit diesen entsprechen, werden durch Gensynthese erzeugt und stellen gegenüber den somatisch hypermutierten Rezeptoren einen homologen Rezeptortyp dar.

Unter "biologischen Bindemolekülen" werden vorliegend beispielsweise, aber nicht ausschließlich, Antikörper inklusive Fusionsproteinen verstanden. Vorteilshafterweise, und daher bevorzugt, ist ein solcher Antikörper ausgewählt aus der Gruppe bestehend aus einem IgG Antikörper, einem IgM Antikörper, einem humanisierten IgG Antikörper, und einem humanen Antikörper, in den die Erkennungssequenz des Epitops eingefügt ist. Ein derartiges Bindemolekül kann auch in Form eines funktionellen Fragments des gesamten Antikörpers bereitgestellt sein, z.B. als Fab-Fragment. Ein Bindemolekül kann auch noch weitere Bereiche umfassen, die z.B. zum Abtöten/Absterben von Neoplasien führen und demgemäß die Funktionalität eines Immuntoxins und/oder Immunzytokins aufweisen (z.B. "Antigen-Drug-Conjugate", ADC). Insbesondere kann ein solches Bindemolekül auch membran- oder zellständig sein. Eine solche membranständige Form eines Bindemoleküls ist z.B. der chimäre Antigenrezeptor ("Chimeric Antigen Receptor"; CAR) auf CAR-T Zellen. Für diagnostische Anwendungen kann das Bindemolekül nachweisbare Markierungen wie insbesondere einen oder mehrere Fluoreszenzfarbstoffe umfassen.

Die Aufgabe des B-Zell Rezeptorkomplexes (BCR) auf der Oberfläche einer B-Zelle ist es, Pathogene zu erkennen und zu binden. Wie bereits erwähnt, führt diese Bindung zu einer Konformationsänderung des BCRs, wodurch eine Signalkaskade ausgelöst wird, die letzten Endes zu einer Aktivierung der B-Zelle führt. Da der Prozess der Generierung eines solchen BCRs auf einer zufälligen Zusammenlagerung von Gensegmenten basiert, kann es vorkommen, dass der neu entstandene BCR unerwünscht körpereigene Strukturen erkennt und so unerwünschterweise "daueraktiviert" wird. Um die Entstehung eines derart "dauerhaft aktiven oder aktivierten" BCRs zu verhindern, existieren verschiedene körpereigne Schutzmechanismen. Werden diese allerdings auf Grund einer krankhaften Veränderung der sich entwickelnden B-Zelle überwunden, kann sich daraus eine maligne oder auch autoimmun manifestierende Erkrankung entwickeln. In anderen Fällen kann es durch die Exposition mit einem Antigen zur Reifung von B-Zellen mit unerwünschten BCR-Eigenschaften kommen.

Während der Reifung der B-Zellen kommt es durch die Exposition der B-Zellen mit dem Antigen zu einem als somatische Hypermutation genannten Effekt, bei dem die in der Keimbahn vorgegebene Variabilität des BCR durch enzymatische Aktivität erhöht wird. Dabei werden zufällige Änderungen in der DNA Sequenz des BCR eingefügt und selektioniert. Als Folge davon können Antikörper/BCR mit höherer Bindestärke an ihr Epitop gebildet werden. Diese Antikörper/BCR sind dadurch einzigartig. Da die somatische Hypermutation(en) aus einem bereits funktionellen BCR hervorgehen, kann es durch diesen Vorgang zur Bildung von sowohl monoklonalen wie auch oligoklonalen B-Zell Populationen kommen. Bei diesen B-Zellen stellt die Aminosäuresequenz des BCR eine Grundlage dar, die durch die Mutationen variiert werden. Diese Variationen können nun durch geeignete Bindemoleküle (Antikörper und deren Fragmente) selektiv erkannt werden. Somit können krankheitsassoziierte B-Zellen spezifisch erkannt (ggf. diagnostiziert) und therapiert werden. Eine Erkennung als solche kann z.B. mittel Durchflusszytometrie erfolgen. Wenn dabei mehre BCR Varianten, die alle einen gemeinsamen Ursprung haben (also oligoklonal sind), verwendet werden, so kann auch ein Antikörper gegen oligoklonale BCR gewonnen werden. Mit anderen Worten werden in diesem Fall als Zielrezeptoren mehrere, unterschiedlich somatisch hypermutierte B-Zell Rezeptoren in Form oligoklonaler Abkömmlinge gleichen Ursprungs eingesetzt.

Eine Therapie kann beispielsweise mittels therapeutischer Antikörper erfolgen (humane, bzw. humanisierte Antikörper, Immuntoxine). Der Vorteil einer derart gestalteten Therapie ist, dass nur der krankmachende Teil der B-Zell Population erfasst wird, wodurch die nach herkömmlichen Therapieansätzen üblicherweise auftretenden systemischen Wirkungen der Therapie minimiert oder sogar vollständig ausgeschlossen werden können.

Im Rahmen der zahlreichen für die vorliegende Erfindung durchgeführten Experimente stellte sich jedoch überraschenderweise heraus, dass Antikörper mit besonderer Spezifität für diese modifizierten Rezeptorbereiche (Epitope) nicht mit üblichen Standardmethoden hergestellt und selektioniert werden können. Erst nachdem die experimentellen Bedingungen derart angepasst wurden, dass im Rahmen von Bindungsstudien gentechnisch veränderte Zellen eingesetzt wurden, deren modifizierte B-Zell Rezeptoren sich in einem nativen und/oder aktivierten bzw. aktivierbaren Zustand befanden, konnten geeignete Antikörper mit der gewünschten und erforderlichen Spezifität gewonnen werden. Mit anderen Worten ist es für die erfindungsgemäß vorgeschlagenen Lösungen von essenzieller Bedeutung, dass die in Bindungsstudien zur Selektion geeigneter prophylaktischer oder therapeutischer sowie diagnostischer Antikörper eingesetzten Zellen ihre modifizierten Bereiche (Epitope) in weitgehend nativer und aktivierbarer Form präsentieren. Hierbei zeigte es sich, dass sog. pro-/prä-B-Zellen aufgrund ihrer physiologischen Konstitution besonders geeignet sind. Die Bereitstellung derartig spezifischer Antikörper und funktioneller Fragmente derselben, die ebenfalls über dieses spezifische Bindungsverhalten verfügen, ermöglicht somit eine Krankheitsspezifische Behandlung, die durch einen deutlich verbesserten Behandlungserfolg und, dank der Reduktion von unerwünschten systemischen Wirkungen, einen deutlich erhöhten Therapieerfolg gekennzeichnet ist. Im Rahmen diagnostischer Anwendungen bedeutet die Möglichkeit des Einsatzes derartig spezifischer Antikörper eine sehr viel genauere Analyse mit einer sehr viel höheren Signifikanz hinsichtlich der Evaluierung eines zu bewertenden Zustandes eines Patienten.

Die Differenzierung von B-Lymphozyten (B-Zellen) z.B. in der Maus kann in voneinander getrennte Phasen unterteilt werden, die phänotypisch und genotypisch weiter unterteilt werden können. Die frühesten B-Vorläufer (pro-B-Zellen), die im Knochenmark von Wildtypmäusen gefunden werden können, stammen von einer pluripotenten hämatopoetischen Stammzelle ab, die keine Zelllinien-spezifischen Marker exprimiert, die aber durch die Expression des Stammzellen Antigens 1 (Sca-1) auf ihrer Oberfläche gekennzeichnet ist.

Im Übergang von der pro-B-Zelle zur prä-B-Zelle werden Zellen generiert, die in der IgH-Kette DJ_{H}-Rearrangements aufweisen (pro-/prä-B-Zellen). Für dieses Gen-Rearragement werden die Gene RAG1 und RAG2 benötigt. In dieser Phase befinden sich sämtliche anderen Ig-Genloci gewöhnlich immer noch in der Keimbahn (GL)-Konfiguration. Prä-B-Zellen mit einem DJ_{H}-Rearrangement werden als prä-B-I-Zellen bezeichnet und können unter spezifischen Bedingungen in Gewebekultur gezogen werden. Das nächste Ereignis eines Rearrangements in diesen Zellen beinhaltet das Rearrangement eines der V_{H}-Gensegmente mit einem vorher existierenden DJ_{H}-Element. Sofern hierdurch ein produktives Rearrangement irgendeines Allels erreicht wird, so dass eine µH-Kette exprimiert werden kann, erhalten diese Zellen ein proliferatives Signal, wodurch sie sich vermehren und zu prä-B-II-Zellen differenzieren. Diese Zellen können den Oberflächenmarker c-kit nicht mehr exprimieren, erlangen aber die Fähigkeit zur Expression von CD25. Ein weiteres produktives Rearrangement auf irgendeinem der IgL-Allele führt zur Differenzierung in unreife B-Zellen, die membrangebundene Immunglobuline (IgM) exprimieren. Diese unreifen nativen B-Zellen können das Knochenmark verlassen und in periphere Lymphoidorgane einwandern, wo sie schließlich auf Antigene treffen können. Im Verlauf einer Immunreaktion können diese B-Zellen in Antikörper-sekretierende Plasmazellen differenzieren, währenddessen es zu einem weiteren DNA-Rekombinationsprozess (Class-Switch-Rekombination) im Locus der konstanten IgH-Region kommen kann, was zu einer Veränderung des von den Plasmazellen sekretierten Antikörperisotyps führt. Einige Erkrankungen sind mit B-Zellen assoziiert, die neben einer Affinitätsreifung auch einen sogenannten Klassenwechsel ("Class Switch Recombination", CSR) durchlaufen haben.

Als Klassenwechsel (CSR = "Class Switch Recombination" oder "Isotype Switching") bezeichnet man in der Immunologie einen Isotypenwechsel bei den B-Zellen des Immunsystems. Im Laufe einer Immunantwort werden unterschiedliche Isotypen der Immunglobuline auf den B-Zellen gebraucht. Durch den Klassenwechsel können B-Zellen ihren Antikörper-Isotyp wechseln. In der VDJ-Sequenz der schweren Kette wird von einer C-Region zu einer anderen, stromabwärts liegenden, C-Region gewechselt. Der Klassenwechsel findet vor allem in den Keimzentren der Lymphknoten statt.

Dieser Wechsel findet immer ausgehend von IgM und IgD hin zu IgG, IgA und IgE statt.

Es ist bekannt, dass der Isotyp die Bindeeigenschaft eines Antikörpers beeinflusst, also einen Einfluss auf die dreidimensionale Struktur des Antikörpers ausübt. Diese Unterschiede werden erfindungsgemäß für die Selektion von spezifischen Antikörpern genutzt.

Ein bevorzugter Anwendungsbereich für die Selektion von Antikörpern gegen CSR-BCRs liegt darin, dass neben der Keimbahnversion auch eine IgM-Version im Rahmen der Selektion bereitgestellt werden. Verfahren zum Erstellen von BCRs des Typs IgM sind im Stand der Technik bekannt.

Wie bereits erwähnt, wird durch die vorliegende Erfindung ein Verfahren zur Herstellung (Identifizierung und Selektion) biologischer Bindemoleküle in Form von Antikörpern oder funktionellen Fragmenten derselben bereitgestellt, wobei die Bindemoleküle selektiv an bestimmte Epitope aktiver oder aktivierbarer membranständiger Rezeptoren oder Rezeptorkomplexe von B-Zellen binden, die die somatische Hypermutation bereits (ganz oder teilweise) durchlaufen haben. Dabei erfolgt die Selektion der gewünschten Bindemoleküle mit selektiver Spezifität für die hypermutierten BCR-Bereiche über eine vergleichende Analyse des Bindungsverhaltens von in den Selektionsansatz eingesetzten Bindemolekülen (Kandidaten) gegenüber einem gegebenen Rezeptor mit hypermutierten Bereichen in Abgrenzung gegenüber dem entsprechenden (korrespondierenden) rekombinant erzeugten Rezeptor des in der Keimbahnkonfiguration (Keimbahntyp), der dem Zustand des BCR vor der während seiner Reifung stattfindenden somatischen Hypermutation entspricht, wobei für die vergleichende Analyse bzw. für die Selektion beide Rezeptortypen (hypermutiert/Keimbahnkonfiguration) in weitgehend nativer Form, d.h. auf der Oberfläche geeigneter B-Zellen (TKO-Zellen im prä-pro-Stadium) exprimiert, als Selektionsplattform bereitgestellt werden. Sofern Bindemoleküle wie insbesondere Antikörper gegen eine oligoklonal gemischte BCR-Population selektiert werden sollen, müssen zur Selektion mehrere verschiedene BCR dieser Population verwendet werden, wobei der gewünschte Antikörper durch seine spezifische Bindung an alle BCR dieser Population gekennzeichnet ist. Mit anderen Worten werden in diesem Fall als Zielrezeptoren mehrere, unterschiedlich somatisch hypermutierte B-Zell Rezeptoren in Form oligoklonaler Abkömmlinge gleichen Ursprungs eingesetzt.

Nach einer weiteren Ausführungsform wird die Selektionsplattform nicht nur zur Identifizierung von aktiven und/oder aktivierbaren Rezeptoren bzw. von an diese spezifisch bindende Antikörper verwendet, sondern auch um Antikörper zu selektionieren, die in der Lage sind, einen aktivierten BCR zu inaktivieren bzw. die Aktivierung eines BCR zu verhindern. Dazu wird der zu untersuchende Antikörper zusammen mit einem Agonisten des BCR (Antigen) auf das bindungsspezifische Signal hin untersucht (s. Beispiel 2). Wenn der BCR nach in Kontakt bringen mit dem Agonisten ein Signal liefert, dieses aber aufgrund einer Vorinkubation mit einem Antikörper (Hybridomüberstand) nicht mehr geliefert wird, so weist der Antikörper antagonistische Eigenschaften auf. Kommt es nach Vorinkubation mit dem Agonisten zu einer Auslöschung des BCR Signals, dann ist der Antikörper in der Lage, die konformationsvermittelte Signaltransduktion zu hemmen. Ein solcher Antikörper wäre eine besondere Art des Antagonisten, da er nicht die Antikörper-Antigen Bindung hemmt, sondern einen anderen Mechanismus der Interaktion aufweist und dadurch in der Lage ist, auch voraktivierte oder konstitutiv aktive BCR zu inhibieren.

Beispiele für Erkrankungen, bei denen mono- bzw. oligoklonale B-Zellen vorliegen, umfassen z.B. aber nicht ausschließlich Leukämien (z.B. CLL) und Autoimmunerkrankungen (MS, rheumatoide Arthritis, Diabetes Typ-1, Zöliakie).

Eine Heilung ist bisher nur durch radikale Zerstörung des Immunsystems (mit anschließender Stammzelltransplantation) möglich. Dieses Verfahren ist aber derart gefährlich, dass es nur in sehr schweren Fällen angewandt wird. Ein Ende der Autoimmunreaktion (bei fortbestehender Autoimmunität) lässt sich erreichen, indem das Antigen vollständig operativ entfernt wird, was aber nur bei Organen infrage kommt, deren Funktion entbehrlich ist oder ersetzt werden kann. Beim Typ-1-Diabetes gelingt der Autoimmunreaktion selbst die vollständige Beseitigung des Antigens (insulinproduzierende β-Zellen), therapiert wird nur der Funktionsverlust (durch Insulingabe).

Nachfolgend wird das Testsystem näher erläutert.

Die EP 3 543 697 beschreibt eine Selektionsplattform zur Selektion autonom aktiver BCR mit der Hilfe genetisch modifizierter prä-pro-B-Zellen (TKO-Zellen). Dieses System erlaubt es, BCR nativ so zu exprimieren, dass Antikörper gegen diese BCR mit einer bisher unerreichten Spezifität und Selektivität selektioniert werden können. Während dieses System zur Verwendung von autonom aktiven BCR erstellt wurde, erlauben es Änderungen in der Anwendung dieses Systems, eine Erweiterung auf das Screening von weiteren BCR (die nicht zwingenderweise autonom aktiv sein müssen). Da das Selektionssystem in der Lage ist, Antikörper, die sich in nur einer Punktmutation von dem "Keimbahntyp" unterscheiden, zu selektionieren, ergibt sich somit die Möglichkeit, die Selektionsplattform, die bisher nur auf autonom aktive BCR ausgelegt war, umfassend zu erweitern. Während bei der EP 3 543 697 der Vergleich zwischen autonom aktiven und nicht autonom aktiven BCR das zentrale Element darstellt, betrifft die hier vorliegende Erfindung den Vergleich zwischen dem BCR nach der somatischen Hypermutation und einem gentechnisch erzeugten "Wildtyp" des BCR, der dem BCR vor Einsetzten der Hypermutation entspricht. Der entsprechende "Wildtyp" BCR wird ausgehend von der Sequenz des hypermutierten BCR erzeugt. Dabei wird zuerst der hypermutierte BCR isoliert und sequenziert und dann aufgrund von Sequenzvergleichen mit den genomischen Komponenten des BCR ein BCR erzeugt, wie er vor der somatischen Hypermutation vorlag. Diese rechnerisch erzeugte Sequenz wird dann mittels Gensynthese erzeugt. Dieser synthetische BCR wird dann ebenfalls in TKO-Zellen exprimiert. Der Vergleich der Bindung von Hybridomüberständen oder generell der erhaltenen Antikörper (vor oder nach eventueller Aufreinigung) ermöglicht es, Antikörper zu erkennen, die spezifisch an die minimalen (aber funktionellen) Änderungen, die durch die Hypermutation entstanden sind, binden. Ein Vergleich von Keimbahntyp vs Mutante kann z.B. mittels Durchflusszytometrie erfolgen. Dabei ist das System in der Lage, minimale Unterschiede bis hin zu einer Aminosäure Unterschied zu erkennen. Als Beispiel demonstriert die Erzeugung eines Antikörper gegen die R110 Mutation des BCR bei CLL die Methode. Es ist jedoch auch möglich, diesen Prozess zu vereinfachen und auf die Kontrollen zu verzichten. Dabei werden dann nur TKO-Zellen mit dem somatisch hypermutierten BCR mit TKO-Zellen des homologen Keimbahntyps verglichen. Dem Fachmann ist jedoch bekannt, dass eine solche Vorgehensweise nicht gleich gute Ergebnisse liefert, als wenn das Verfahren die Verwendung von Kontrollen umfasste.

Einzelne Aspekte der vorliegenden Erfindung werden nachfolgend anhand von Beispielen näher erläutert.

### Beispiel 1

Aus Studien zur Genetik der CLL ist die Aminosäuresequenz des mutierten Rezeptors bekannt. Er besteht aus zwei Aminosäureketten, nämlich der leichten Kette (LC) und der schweren Kette (HC).
R110 HC: SEQ ID NO 1
R110 LC: SEQ ID NO 02

Der entsprechende Keimbahntyp BCR hat die Sequenz
WT HC: SEQ ID NO 03
WT LC: SEQ ID NO 04

Beide Sequenzen unterscheiden sich in diesem Fall durch mehr als nur eine Aminosäure.

Den Ausgangspunkt für die Herstellung von Triple-Knockout-Zellen (TKO) bilden transgene Mäuse, welche einen jeweiligen Knockout für die Gene Lambda5, RAG2 und SLP65 aufweisen (Dühren von Minden et al., 2012, Nature 489, p309-313). Die Erstellung solcher Mäuse ist dem Fachmann bekannt und gehört zum Stand der Technik. Für die Gewinnung der Zellen wurde den Mäusen nach ihrer Tötung das Knochenmark der Oberschenkelknochen extrahiert. Die so gewonnenen Zellen wurden anschließend unter Bedingungen in Kultur genommen, die das Überleben von pro-/prä-B-Zellen begünstigen (37°C, 7,5% CO₂, Iscoves Medium, 10% FCS, P/S, murinem IL7). Nach mehreren Passagen wurden zur Kontrolle eine FACS-Sortierung durchgeführt, die pro-/prä-B-Zellen Zellen sortiert und anschließend wieder in Kultur genommen. Die dazu verwendeten Marker sind dem Fachmann bekannt.

Für die Rekonstitution mit einem 'BCR of Interest' wurden die entsprechenden, für die schweren (HC) und leichten (LC) Ketten kodierenden Sequenzen synthetisiert und dann jeweils in einen CMV-Promotor aufweisende Expressionsvektoren kloniert. Diese wurden mittels Lipofektion in die Verpackungszelllinie (Phoenix-Zelllinie) eingebracht. Nach einer 36 Stunden andauernden Inkubation wurde der Virusüberstand abgenommen und für eine Spinfektion der TKOs verwendet. Sowohl die Arbeiten zur Gewinnung der Überstände als auch die Spinfektion der TKO sind weitläufig bekannte Verfahren und dem Fachmann bekannt. Es wird darauf hingewiesen, dass zur Ausführung der vorliegenden Selektionsverfahren nicht unbedingt TKO-Zellen eingesetzt werden müssen, da der 'Knockout' des Gens SLP65 lediglich für besondere Ausführungsformen, bei denen die Aktivierung des Rezeptors nachgewiesen werden soll, benötigt wird. Mit anderen Worten kann die vorliegende technische Lehre auch unter Verwendung von Zellen ausgeführt werden, die lediglich jeweils einen 'Knockout' bezüglich der Gene RAG2 und Lambda5 umfassen.

Die strukturellen Besonderheiten von Subset-2 B-Zell Rezeptoren wurden der entsprechenden Literatur entnommen (s.o.). Exemplarische CLL-R110 VH und vollständige LC DNA-Segmente wurden von einem Lohnhersteller in einem Standardverfahren synthetisiert. Diese wurden dann mit einem murinen IgG1-Konstantensegment mittels PCR fusioniert und in einen CMV-Vektor kloniert. Die Sequenz des fertigen Vektors wurde mittels Sanger-Sequenzierung bestätigt.

Für die Expression des R110 IgG1 (SEQ ID NO 1 und SEQ ID NO 2 - R110G ist ein Bestandteil des BCR des Typs CLL-Subset 2 (CLL-Subset 2 wird u.a. in folgenden Publikationen beschrieben: P. Baliakas et al., 2017, Haematologica 103: e158-e161; B. Stamatopoulos et al., 2018, Clinical cancer research 24.20: 5048-5057)), wurde ein humanes zelluläres Expressionssystem basierend auf HEK293T Zellen genutzt. Für die Transfektion wurde ein auf Polyethylenimin (PEI) basierendes Protokoll angewendet. Nach mehreren Passagen wurde der Überstand gepoolt, und das in dem vereinigten Zellüberstand enthaltende Medium wurde mittels Protein G Säulchen aufgereinigt. Die Reinheit und Qualität des R110 IgG1 wurde mittels Westernblot bestimmt.

Die Herstellung von monoklonalen Antikörpern erfolgte nach dem Standardverfahren in Mäusen und der anschließenden Generierung von Hybridoma-Zellen. Das Screening nach positiven Klonen erfolgte nicht wie herkömmlich mittels ELISA. Da es sich bei der Zielstruktur um einen membranständigen Rezeptor handelt, ist es von zentraler Bedeutung, die Bindung der potentiellen Antikörper auch in einem zellulären System, d.h. unter weitgehender Wahrung der für diesen Zelltyp nativen zellphysiologischen Zustände, zu validieren. Es wurden zunächst Gruppen von gepoolten Überständen mittels FACS-Analyse auf Bindungsereignisse untersucht. Dazu wurden verschiedene CLL-R110 BCR Varianten auf der Oberfläche einer Zelllinie (TKO) exprimiert, welche selber keinen BCR exprimieren kann. So konnten zunächst die Überstände identifiziert werden, deren Antikörper eine Bindung aufzeigten. Anschließend wurden die Überstände der einzelnen Hybridom-Klone hinsichtlich ihrer Bindung eingehender untersucht, um auf diese Weise hochspezifische Klone mit hoher Affinität zu identifizieren.

Für das Screening-Verfahren wurden im Rahmen der vorhergehenden Transformation unterschiedliche Vektoren für die folgenden Kombinationen aus schwerer Kette (HC) und leichter Kette (LC) der entsprechenden CLL-BCRs eingesetzt, wobei diese Kombinationen auf der Oberfläche des BCR-Rekonstitutionssystems verwendet wurden:
- Kontrolle (Transformationsvektor ohne BCR)
- Vektor mit für den CLL-R110G BCR kodierender DNA
- Vektor mit für den Keimbahntyp BCR zu R110G kodierender DNA
- Vektor mit für den CLL-R110G typischer HC / Keimbahntyp LC

In der 1. Selektionsrunde wurden Überstände mehrerer Klone vereinigt und hinsichtlich ihres Bindungsprofils an die Selektionsmatrix untersucht. Ein positives Bindungsprofil ist gegeben, wenn sich eine spezifische Bindung an den "BCR-of-Interest" zeigt. Gruppen, die ein solches Profil zeigten, wurden vereinzelt, und das Bindungsprofil der einzelnen Klone wurde im Rahmen einer zweiten Selektionsrunde nochmals auf der Selektionsmatrix charakterisiert. Die Bindung der monoklonalen Antikörper wurde unter Anwendung eines FACS-Bindungsassays unter Verwendung eines Fluoreszenz-markierten Anti-Mouse-IgG Antikörpers verifiziert, wobei verschiedene B-Zellen mit folgender Spezifität eingesetzt wurden: A) kein BCR (Kontrolle); B) ein CLL-R110G BCR; C) ein BCR des Keimbahntyps zu CLL-R110G; D) ein BCR mit einer CLL-R110G typischen schweren Kette und leichten Kette des Keimbahntyps zu CLL-R110G.

Aufgrund des Befundes, dass der Antikörper nur an die Zellen mit den Zielstrukturen bindet (CLL-R110G BCR), kann gefolgert werden, dass hier ein Antikörper vorliegt, der spezifisch an Zellen mit dem mutierten Rezeptor bindet.

Hierbei zeigte sich, dass die Verwendung von Zellen, die sich im pro-/prä-Stadium der B-Zell Entwicklung befinden, für die zum Nachweis benötigte exakte Expression des BCR notwendig ist. Diese Zellen sind entwicklungsgenetisch eingerichtet, um neue BCR durch exakte Faltung und Expression auf ihrer Oberfläche darzustellen. Durch die Inaktivierung (Knockout) von RAG2 und Lambda5 wird die Expression eines endogenen BCR bzw. prä-BCR verhindert. Die Deletion von SLP65 und die anschließende Rekonstruktion eines induzierbaren SLP65 ermöglicht es, das Aktivitätsniveau des "BCR of Interest" zu charakterisieren, was z.B. durch Zugabe eines Antigens erfolgen kann.

Zur Bestimmung der Aminosäurensequenz der mittels Selektion ausgewählten monoklonalen Antikörper wurde aus den einzelnen Hybridom-Klonen die mRNA isoliert, daraus cDNA generiert und diese mittels Anchor-PCR amplifiziert (Rapid expression cloning of human immunoglobulin Fab fragments for the analysis of antigen specificity of B cell lymphomas and anti-idiotype lymphoma vaccination; Osterroth F, Alkan O, Mackensen A, Lindemann A, Fisch P, Skerra A, Veelken H., J Immunol Methods 1999 Oct 29; 229(1-2):141-53).

Nach Identifizierung und Sequenzbestimmung der für die Bindung wichtigen Bereiche (CDRs) wurden diese mittels PCR auf ein humanes Antikörpergerüst übertragen. Dazu wurde die VH-Sequenz aus den humanen FR-Regionen und den murinen CDR Regionen *in silico* generiert und anschließend als DNA-Fragmente synthetisiert. Diese wurden anschließend mittels PCR mit einem humanen IgG1 fusioniert und in einen für die Expression geeigneten Vektor kloniert.

Für die Generierung der monoklonalen Antikörper wurden neben den kompletten Immunglobulinen auch synthetische Peptide verwendet, welche die Regionen für die Fähigkeit eines autonomen Signals repräsentierten.

Der spezifische monoklonale Antikörper gegen R110G BCR wurde sequenziert. Dabei wurden die folgenden Aminosäurequenzen bestimmt, wobei die SEQ ID NO. 5 den variablen Teil der schweren Kette (HC), und die SEQ ID NO. 6 den variablen Teil der leichten Kette (LC) betreffen, und wobei die markierten Bereiche - in der angegebenen Reihenfolge - CDR 1, 2 und 3 bezeichnen.
SEQ ID NO. 5 (AVA-mAb01 HC)
SEQ ID NO. 6 (AVA-mAb01 LC)

Die mit CDR1, CDR2 und CDR3 korrespondierenden Teilsequenzen der schweren Kette gemäß SEQ ID NO. 5 sind in den SEQ ID NOS. 7 bis 9 angegeben, während die mit CDR1, CDR2 und CDR3 korrespondierenden Teilsequenzen der leichten Kette gemäß SEQ ID NO. 6 in den SEQ ID NOS. 10 bis 12 dargestellt sind.
SEQ ID NO. 7 (AVA-mAB01 CDR1 HC)
   GFSLTSYG
SEQ ID NO. 8 (AVA-mAB01 CDR2 HC)
   IWRGGGT
SEQ ID NO. 9 (AVA-mAB01 CDR3 HC)
   ARSRYDEEESMNY
SEQ ID NO. 10 (AVA-mAB01 CDR1 LC)
   GNIHSY
SEQ ID NO. 11 (AVA-mAB01 CDR2 LC)
   NAKT
SEQ ID NO. 12 (AVA-mAB01 CDR3 LC)
   QHFWNTPPT

### Beispiel 2

Es wurden die gleichen Hybridome wie in Beispiel I verwendet. Nur das Verfahren der Selektion unterscheidet sich. Die Selektion fand in diesem Falle unter Verwendung von TKO-Zellen statt, die zuvor zur Exposition mit dem Antigen des BCR (5µg/ml, 5 Minuten) inkubiert wurden. Dadurch kam es zur Bindung des Antigens an den BCR. Eine Aktivierung des BCR (mit eventueller Internalisierung des BCR) findet nicht statt, da SLP65 noch nicht induziert ist. Die Zellen wurden dann aufgeteilt. Ein Teil wurde zur Funktionsüberprüfung in einem BD Fortessa II FACS Gerät gemessen. Dieses Gerät erlaubt die Messung zu unterbrechen und mit neuen Parametern wieder aufzunehmen. Die meisten anderen FACS Geräte erlauben dies nicht. Dazu wurden die Zellen 45 Minuten lang vor der Messung mit Indo-1 gemäß Herstelleranweisung inkubiert. Indo-1 erzeugt bei Calciuminflux in die Zelle ein Fluoreszenzsignal, das dann vom FACS Gerät gemessen werden kann. Die Zellen wurden für eine Minute gemessen, um eine Baseline zu erzeugen. Dann wurde 4-OH-Tamoxifen zugegeben (2mM), wodurch die Signaltransduktion des BCR rekonstitutiert wurde, und es konnte ein Calciumsignal detektiert werden. Dieses Calciumsignal zeigte die Aktivierung des BCR an. Um bei einem BCR die Aktivierung (bzw. die Inaktivierung) nachzuweisen, musste das Calciumsignal des aktivierbaren Rezeptors (in Zellen mit rekonstituiertem Calciumsignal) mit dem Signal des gleichen BCR in Zellen ohne rekonstituiertes Calciumsignal verglichen werden. Dabei wurde das Ca-Signal vor und nach der Aktivierung mit dem Agonisten verglichen. Nachdem die Aktivierung der Zellen nachgewiesen werden konnte, wurde nun die zweite Charge der Zellen (ohne 4-OHT Aktivierung) zur Selektion eines geeigneten Antikörpers gegen den aktivierte BCR verwendet (siehe Beispiel 1). Wenn der dann selektionierte Antikörper an nicht aktivierte BCR nicht bindet, so ist erfolgreich ein Antikörper, der nur an aktive BCR bindet, selektioniert worden.

### Beispiel III

Anschließend wurden die Zellen vor der Stimulation mit dem potentiellen Inhibitor inkubiert (5µg/ml, 5 Minuten). Diese Zellen wurden dann mit dem Agonisten (Antigen siehe Beispiel 2) stimuliert (bzw. bei autonom aktiven Zellen ohne Stimulation verwendet, da das Stimulans sich auf der Zelle selbst befindet), und das Ca-Signal nach der Hemmung mit dem Inhibitor wurde mit dem Ca-Signal der Zellen ohne Inhibitor verglichen. Eine Inhibition bedeutete, dass das CA-Signal der Zellen mit Inhibitor deutlich geringer war (mindestens 50% des Netto-Vergleichsignals).

Zum Screening in diesem Falle wurden verwendet: (induziert bedeutet, dass diese Zellen zuvor mit Hydroxy-Tamoxifen behandelt wurden)
- Induzierte Kontrolle (Transformationsvektor ohne BCR)
- Induzierte Zellen: Mit Vektor mit für den CLL-R110G BCR kodierender DNA transformiert
- Induzierte Zellen: Mit Vektor mit für den Keimbahntyp BCR zu R110G kodierender DNA transformiert

Bei BCR, die nicht autonom aktiv sind, wurde zusätzlich noch der Schritt der Vorinkubation mit dem Agonisten benötigt. Ansonsten wurden dieselben Ansätze verwendet.

## Patentansprüche

1. Verfahren zur Selektion eines biologischen Bindemoleküls, das spezifisch an einen somatisch hypermutierten B-Zell Rezeptor als Zielrezeptor, nicht jedoch an einen homologen nicht-hypermutierten B-Zell Rezeptor bindet, im Rahmen eines zellbasierten Systems unter Verwendung von unreifen B-Zellen, die sich im pro-/prä-Stadium befinden, umfassend die folgenden Schritte:
(a) Bereitstellung einer Mehrzahl biologischer Bindemoleküle, die durch Immunisierung eines Säugers mit B-Zell Rezeptoren oder deren Fragmenten sowie anschließende Immortalisierung und Aufreinigung gewonnen wurden;
(b) Bereitstellung von unreifen B-Zellen im pro-/prä-Stadium, die nicht in der Lage sind, die nativen Gene für RAG2 und/oder RAG1 sowie Lambda5 zu exprimieren, die aber in die Lage versetzt wurden, auf ihrer Zelloberfläche somatisch hypermutierte B-Zell Rezeptoren als Zielrezeptoren zu exprimieren;
(c) Bereitstellung von unreifen B-Zellen im pro-/prä-Stadium, die nicht in der Lage sind, die nativen Gene für RAG2 und/oder RAG1 sowie Lambda5 zu exprimieren, die aber in die Lage versetzt wurden, auf ihrer Zelloberfläche rekombinant hergestellte B-Zell Rezeptoren, die im Vergleich zu den Rezeptoren gemäß (b) homologe, jedoch keine hypermutierten Bereiche aufweisenden Rezeptoren sind, als Referenzrezeptoren zu exprimieren;
(d) Vergleichende Untersuchung des Bindungsverhaltens der gemäß Schritt (a) bereitgestellten Bindemoleküle gegenüber Zellen, die gemäß der Schritte (b) und (c) bereitgestellt werden;
(e) Selektion mindestens eines Bindemoleküls, das spezifisch an gemäß Schritt (b) bereitgestellte Zellen, nicht jedoch an gemäß Schritt (c) bereitgestellte Zellen bindet.

2. Verfahren nach Anspruch 1, **dadurch gekennzeichnet, dass** der Zielrezeptor ein aktivierbarer B-Zell Rezeptor ist.

3. Verfahren nach Anspruch 1, **dadurch gekennzeichnet, dass** die gemäß der Schritte (b) und (c) bereitgestellten Zellen zusätzlich nicht in der Lage sind, das native Gen für SLP65 zu exprimieren.

4. Verfahren nach einem der vorhergehenden Ansprüche, **dadurch gekennzeichnet, dass** als Zielrezeptoren mehrere, unterschiedlich somatisch hypermutierte B-Zell Rezeptoren in Form oligoklonaler Abkömmlinge gleichen Ursprungs eingesetzt werden.

5. Verfahren nach einem der vorhergehenden Ansprüche, **dadurch gekennzeichnet, dass** der Zielrezeptor zuvor mit einem Antigen aktiviert wurde.

6. Verfahren nach Anspruch 5, **dadurch gekennzeichnet, dass** die erfolgreiche Aktivierung vor der Selektion bestätigt wurde.

7. Verfahren nach Anspruch 3, **dadurch gekennzeichnet, dass** Schritt (e) neben der Feststellung einer spezifischen Bindung des Bindemoleküls an gemäß Schritt (b) bereitgestellte Zellen eine Bestätigung durch eine Aktivitätsmessung nach Induktion von SLP65 einschließt.

## Claims

1. Method for selecting a biological binding molecule which specifically binds to a somatically hypermutated B-cell receptor as the target receptor, but not to a homologous, non-hypermutated B-cell receptor, in a cell-based system using immature B cells, which are in the pro/pre stage, comprising the following steps:
(a) providing a plurality of biological binding molecules obtained by immunizing a mammal with B-cell receptors or their fragments and then immortalizing and purifying them;
(b) providing immature B cells in the pro/pre stage, which are not able to express the native genes for RAG2 and/or RAG1 as well as Lambda5, but which have been enabled to express somatically hypermutated B-cell receptors as target receptors on their cell surface;
(c) providing immature B cells in the pro/pre stage, which are not able to express the native genes for RAG2 and/or RAG1 as well as Lambda5, but which have been enabled to express recombinantly produced B-cell receptors on their cell surface, which are homologous receptors compared to the receptors according to (b), but not having hypermutated regions, as reference receptors;
(d) comparatively investigating the binding behavior of the binding molecules provided according to step (a) with respect to cells that are provided according to steps (b) and (c);
(e) selecting at least one binding molecule which specifically binds to cells provided according to step (b), but not to cells provided according to step (c).

2. Method according to claim 1, **characterized in that** the target receptor is an activatable B-cell receptor.

3. Method according to claim 1, **characterized in that** the cells provided according to steps (b) and (c) are additionally unable to express the native gene for SLP65.

4. Method according to any of the preceding claims, **characterized in that** a plurality of differently somatically hypermutated B-cell receptors in the form of oligoclonal derivatives of the same origin are used as target receptors.

5. Method according to any of the preceding claims, **characterized in that** the target receptor was previously activated with an antigen.

6. Method according to claim 5, **characterized in that** the successful activation was confirmed before the selection.

7. Method according to claim 3, **characterized in that** step (e), in addition to determining a specific binding of the binding molecule to cells provided according to step (b), includes a confirmation by an activity measurement after induction of SLP65.

## Revendications

1. Procédé de sélection d'une molécule de liaison biologique qui se lie spécifiquement à un récepteur de lymphocyte B à hypermutation somatique en tant que récepteur cible, mais pas à un récepteur de lymphocyte B homologue sans hypermutation, dans le cadre d'un système à base de cellules en utilisant des lymphocytes B non matures, qui se trouvent au stade pro-/prélymphocytaire, comprenant les étapes suivantes :
(a) fournir une multitude de molécules de liaison biologique, qui ont été obtenues par immunisation d'un mammifère avec des récepteurs de lymphocyte B ou leurs fragments ainsi que l'immortalisation et la purification consécutives ;
(b) fournir des lymphocytes B non matures au stade pro-/prélymphocytaire, qui ne sont pas en mesure d'exprimer les gènes natifs pour RAG2 et/ou RAG1 et Lambda5, qui ont été cependant mis en capacité d'exprimer, à leur surface cellulaire, des récepteurs de lymphocyte B à hypermutation somatique en tant que récepteurs cibles ;
(c) fournir des lymphocytes B non matures au stade pro-/prélymphocytaire, qui ne sont pas en mesure d'exprimer les gènes natifs pour RAG2 et/ou RAG1 et Lambda5, qui ont été cependant mis en capacité d'exprimer, à leur surface cellulaire, des récepteurs de lymphocyte B préparés par voie recombinante, qui sont des récepteurs homologues, ne présentant cependant pas de régions à hypermutation, par rapport aux récepteurs selon (b), en tant que récepteurs cibles ;
(d) analyser comparativement le comportement de liaison des molécules de liaison fournies selon l'étape (a) par rapport à des cellules qui sont fournies selon les étapes (b) et (c) ;
(e) sélectionner au moins une molécule de liaison qui se lie spécifiquement aux cellules fournies selon l'étape (b), mais pas aux cellules fournies selon l'étape (c).

2. Procédé selon la revendication 1, **caractérisé en ce que** le récepteur cible est un récepteur de lymphocyte B activable.

3. Procédé selon la revendication 1, **caractérisé en ce que** les cellules fournies selon les étapes (b) et (c) ne sont en plus pas en mesure d'exprimer le gène natif pour SLP65.

4. Procédé selon l'une des revendications précédentes, **caractérisé en ce que** plusieurs récepteurs de lymphocyte B à hypermutation somatique différente sont mis en œuvre sous la forme de dérivés oligoclonaux de même origine.

5. Procédé selon l'une des revendications précédentes, **caractérisé en ce que** le récepteur cible a été activé auparavant avec un antigène.

6. Procédé selon la revendication 5, **caractérisé en ce que** l'activation réussie a été confirmée avant la sélection.

7. Procédé selon la revendication 3, **caractérisé en ce que** l'étape (e) inclut, en plus de la constatation d'une liaison spécifique de la molécule de liaison à des cellules fournies selon l'étape (b), une confirmation par une mesure d'activité après induction de SLP65.


### SEQUENCE LISTING

<110> AVA Lifescience GmbH
<120> Verfahren zur Selektion biologischer Bindemoleküle
<130> Prio vom 20.12.2018
<160> 12
<170> BiSSAP 1.3.6
<210> 1
   <211> 221
   <212> PRT
   <213> Homo sapiens
<400> 1
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Arg Ser Tyr
               20                  25                  30
   Ser Met Asn Trp Val Arg Gln Ala Pro Gly Lys Gly Leu Glu Trp Val
           35                  40                  45
   Ser Ser Ile Ile Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Gln Asn Ala Met Asp Val Trp Gly Gln Gly Thr Thr Val
               100                 105                 110
   Thr Val Ser Ser Asp Ser Ala Ser Ala Pro Thr Leu Phe Pro Leu Val
           115                 120                 125
   Ser Cys Glu Asn Ser Pro Ser Asp Thr Ser Ser Val Ala Val Gly Cys
       130                 135                 140
   Leu Ala Gln Asp Phe Leu Pro Asp Ser Ile Thr Phe Ser Trp Lys Tyr
   145                 150                 155                 160
   Lys Asn Asn Ser Asp Ile Ser Ser Thr Arg Gly Phe Pro Ser Val Leu
                   165                 170                 175
   Arg Gly Gly Lys Tyr Ala Ala Thr Ser Gln Val Leu Leu Pro Ser Lys
               180                 185                 190
   Asp Val Met Gln Gly Thr Asp Glu His Val Val Cys Lys Val Gln His
           195                 200                 205
   Pro Asn Gly Asn Lys Glu Lys Asn Val Pro Leu Pro Val
       210                 215                 220
<210> 2
   <211> 246
   <212> PRT
   <213> Homo sapiens
<400> 2
   Ile Arg Ser Leu Glu Ala Thr Met Ala Trp Thr Val Leu Leu Leu Gly
   1               5                   10                  15
   Leu Leu Ser His Cys Thr Gly Ser Val Thr Ser Tyr Glu Leu Thr Gln
               20                  25                  30
   Pro Pro Ser Val Ser Val Ala Pro Gly Lys Thr Ala Arg Ile Thr Cys
           35                  40                  45
   Ala Gly Asn Asn Ile Gly Ser Lys Ser Val His Trp Tyr Gln Gln Lys
       50                  55                  60
   Pro Gly Gln Ala Pro Val Leu Val Ile Tyr Tyr Asp Ser Asp Arg Pro
   65                  70                  75                  80
   Ser Gly Ile Pro Glu Arg Phe Ser Gly Ser Asn Ser Gly Asn Thr Ala
                   85                  90                  95
   Thr Leu Thr Ile Ser Arg Val Glu Ala Gly Asp Glu Ala Asp Tyr Tyr
               100                 105                 110
   Cys Gln Val Trp Asp Ser Gly Ser Asp His Pro Trp Val Phe Gly Gly
           115                 120                 125
   Gly Thr Lys Leu Thr Val Leu Arg Gln Pro Lys Ala Ala Pro Ser Val
       130                 135                 140
   Thr Leu Phe Pro Pro Ser Ser Glu Glu Leu Gln Ala Asn Lys Ala Thr
   145                 150                 155                 160
   Leu Val Cys Leu Ile Ser Asp Phe Tyr Pro Gly Ala Val Thr Val Ala
                   165                 170                 175
   Trp Lys Ala Asp Ser Ser Pro Val Lys Ala Gly Val Glu Thr Thr Thr
               180                 185                 190
   Pro Ser Lys Gln Ser Asn Asn Lys Tyr Ala Ala Ser Ser Tyr Leu Ser
           195                 200                 205
   Leu Thr Pro Glu Gln Trp Lys Ser His Arg Ser Tyr Ser Cys Gln Val
       210                 215                 220
   Thr His Glu Gly Ser Thr Val Glu Lys Thr Val Ala Pro Thr Glu Cys
   225                 230                 235                 240
   Ser Glu Phe Arg Pro Ser
                   245
<210> 3
   <211> 221
   <212> PRT
   <213> Homo sapiens
<400> 3
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gln Asn Ala Met Asp Val Trp Gly Gln Gly Thr Thr Val
               100                 105                 110
   Thr Val Ser Ser Asp Ser Ala Ser Ala Pro Thr Leu Phe Pro Leu Val
           115                 120                 125
   Ser Cys Glu Asn Ser Pro Ser Asp Thr Ser Ser Val Ala Val Gly Cys
       130                 135                 140
   Leu Ala Gln Asp Phe Leu Pro Asp Ser Ile Thr Phe Ser Trp Lys Tyr
   145                 150                 155                 160
   Lys Asn Asn Ser Asp Ile Ser Ser Thr Arg Gly Phe Pro Ser Val Leu
                   165                 170                 175
   Arg Gly Gly Lys Tyr Ala Ala Thr Ser Gln Val Leu Leu Pro Ser Lys
               180                 185                 190
   Asp Val Met Gln Gly Thr Asp Glu His Val Val Cys Lys Val Gln His
           195                 200                 205
   Pro Asn Gly Asn Lys Glu Lys Asn Val Pro Leu Pro Val
       210                 215                 220
<210> 4
   <211> 220
   <212> PRT
   <213> Homo sapiens
<400> 4
   Ser Tyr Val Leu Thr Gln Pro Pro Ser Val Ser Val Ala Pro Gly Lys
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Ile Tyr
           35                  40                  45
   Tyr Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Pro Trp Val Phe Gly Gly Gly Thr Lys Leu Thr Val Leu Arg Gln Pro
               100                 105                 110
   Lys Ala Ala Pro Ser Val Thr Leu Phe Pro Pro Ser Ser Glu Glu Leu
           115                 120                 125
   Gln Ala Asn Lys Ala Thr Leu Val Cys Leu Ile Ser Asp Phe Tyr Pro
       130                 135                 140
   Gly Ala Val Thr Val Ala Trp Lys Ala Asp Ser Ser Pro Val Lys Ala
   145                 150                 155                 160
   Gly Val Glu Thr Thr Thr Pro Ser Lys Gln Ser Asn Asn Lys Tyr Ala
                   165                 170                 175
   Ala Ser Ser Tyr Leu Ser Leu Thr Pro Glu Gln Trp Lys Ser His Arg
               180                 185                 190
   Ser Tyr Ser Cys Gln Val Thr His Glu Gly Ser Thr Val Glu Lys Thr
           195                 200                 205
   Val Ala Pro Thr Glu Cys Ser Glu Phe Arg Pro Ser
       210                 215                 220
<210> 5
   <211> 119
   <212> PRT
   <213> Homo sapiens
<400> 5
   Gln Val Gln Leu Gln Gln Ser Gly Pro Gly Leu Val Gln Pro Ser Gln
   1               5                   10                  15
   Ser Leu Ser Ile Thr Cys Thr Val Ser Gly Phe Ser Leu Thr Ser Tyr
               20                  25                  30
   Gly Ile His Trp Val Arg Gln Ser Pro Gly Lys Gly Leu Glu Trp Leu
           35                  40                  45
   Gly Val Ile Trp Arg Gly Gly Gly Thr Asp Ser Asn Ala Ala Phe Met
       50                  55                  60
   Ser Arg Leu Ser Ile Thr Lys Asp Asn Ser Lys Ser Gln Val Phe Phe
   65                  70                  75                  80
   Lys Met Asn Ser Leu Gln Ala Asp Asp Thr Ala Ile Tyr Tyr Cys Ala
                   85                  90                  95
   Arg Ser Arg Tyr Asp Glu Glu Glu Ser Met Asn Tyr Trp Gly Gln Gly
               100                 105                 110
   Thr Ser Val Thr Val Ser Ser
           115
<210> 6
   <211> 107
   <212> PRT
   <213> Homo sapiens
<400> 6
   Gln Ile Val Leu Thr Gln Ser Pro Ala Ser Leu Ser Ala Ser Val Gly
   1               5                   10                  15
   Glu Thr Val Thr Ile Thr Cys Arg Ala Ser Gly Asn Ile His Ser Tyr
               20                  25                  30
   Leu Ala Trp Tyr Gln Gln Lys Gln Gly Lys Ser Pro Gln Leu Leu Val
           35                  40                  45
   Tyr Asn Ala Lys Thr Leu Ala Asp Gly Val Pro Ser Arg Phe Ser Gly
       50                  55                  60
   Ser Gly Ser Gly Thr Gln Tyr Ser Leu Lys Ile Asn Ser Leu Gln Pro
   65                  70                  75                  80
   Glu Asp Phe Gly Ser Tyr Tyr Cys Gln His Phe Trp Asn Thr Pro Pro
                   85                  90                  95
   Thr Phe Gly Ala Gly Thr Lys Leu Glu Leu Lys
               100                 105
<210> 7
   <211> 8
   <212> PRT
   <213> Homo sapiens
<400> 7
   Gly Phe Ser Leu Thr Ser Tyr Gly
   1               5
<210> 8
   <211> 7
   <212> PRT
   <213> Homo sapiens
<400> 8
   Ile Trp Arg Gly Gly Gly Thr
   1               5
<210> 9
   <211> 13
   <212> PRT
   <213> Homo sapiens
<400> 9
   Ala Arg Ser Arg Tyr Asp Glu Glu Glu Ser Met Asn Tyr
   1               5                   10
<210> 10
   <211> 6
   <212> PRT
   <213> Homo sapiens
<400> 10
   Gly Asn Ile His Ser Tyr
   1 5
<210> 11
   <211> 4
   <212> PRT
   <213> Homo sapiens
<400> 11
   Asn Ala Lys Thr
   1
<210> 12
   <211> 9
   <212> PRT
   <213> Homo sapiens
<400> 12
   Gln His Phe Trp Asn Thr Pro Pro Thr
   1 5